# EUROPEAN PATENT SPECIFICATION

(11) **EP 2 702 925 B1**
(45) Date of publication and mention of the grant of the patent: **19.10.2016**
(21) Application number: 13791348.9
(22) Date of filing: 19.03.2013
(51) Int. Cl.: A61B 1/00, G02B 23/24

(54) **ENDOSCOPE SYSTEM**
ENDOSKOPSYSTEM
SYSTÈME D'ENDOSCOPE

(30) Priority: 14.05.2012 JP 2012110802
(43) Date of publication of application: 05.03.2014
(73) Proprietor: Olympus Corporation, Tokyo 192-8507 (JP)
(72) Inventor: OKANIWA, Suguru, Shibuya-ku, Tokyo 151-0072 (JP)
(74) Representative: Schicker, Silvia
(86) International application number: PCT/JP2013/057864
(87) International publication number: WO 2013/172089

(56) References cited:
- EP-A1- 0 535 847
- WO-A1-2004/037075
- JP-A- H1 142 206
- JP-A- 2002 330 924
- JP-A- 2006 136 743
- US-A1- 2003 122 927
- US-A1- 2004 249 246

## Description

### Technical Field

The present invention relates to an endoscope system including a plurality of endoscopes, including actively bendable first bending portions and second bending portions to be passively bent by external force, and capable of improving operability in inserting the endoscopes into a large intestine or the like.

### Background Art

As it is well known, endoscopes have been widely used for observation, treatment, and the like of an inside of a body (a body cavity) of a living organism and inspection, repair, and the like of an inside of a plant facility for industrial purposes. In particular, an endoscope for medical use has been widely used because it is possible to observe, without requiring dissection, a test target region in a body cavity by inserting an elongated insertion portion into the body cavity and perform curative treatment using a treatment instrument according to necessity. As the endoscope in a medical field, in order to improve insertability into a body cavity of a patient, for example, Japanese Patent Application Laid-Open Publication No. 2007-54400 discloses an endoscope including an active type bending portion and a passive type bending portion that easily bends upon receiving external force.

Incidentally, as the endoscope for medical use, in an insertion process of an insertion portion into a large intestine, which is a body cavity, various models including insertion portions having different outer diameters (thicknesses) are selected as appropriate according to sex, various body shapes, presence or absence of adhesion, and the like of patients. An inserting operation method for the endoscope is different according to an outer diameter of an insertion portion. For example, in the case of a large-diameter endoscope having a large diameter of an insertion portion, operation for inserting the insertion portion while straightening the large intestine by performing twisting operation, traction operation, and the like of the insertion portion is mainly performed. On the other hand, in the case of a small-diameter endoscope having a small outer diameter of an insertion portion, operation for simply pushing and inserting the insertion portion into the large intestine is mainly performed.

However, the conventional large-diameter endoscope having the passive type bending portion has such problems that resilience is easily lost due to bending of the passive type bending portion and the distal end of the insertion portion is hard to be hooked on the bend of the large intestine, which results in difficulty in straitening the large intestine. On the other hand, regardless of whether the endoscope is a large-diameter endoscope or a small-diameter endoscope, it is sometimes preferable that the endoscope includes the passive type bending portion in the insertion portion in order to improve insertability and operability at the time when the endoscope is just pushed into the large intestine.

The present invention has been achieved in view of the above circumstances and an object of the present invention is to provide an endoscope system in which, among models of endoscopes provided with insertion portions having different outer diameters, the insertion portions suitable for insertion operation into a large intestine by the respective models are provided to obtain optimum insertability.

### Disclosure of Invention

### Means for Solving the Problem

An endoscope system according to an aspect of the present invention is an endoscope system including a plurality of endoscopes including insertion portions configured by bending portions and flexible tube portions, outer diameters of at least the flexible tubes being different from one another. Each of the bending portions in the plurality of endoscopes includes a first bending portion subjected to bending operation to actively bend and a second bending portion connected to the first bending portion and passively bent by external force. The flexible tubes are respectively connected to the second bending portion and formed to have rigidity higher than the rigidity of the second bending portion. In the plurality of endoscopes, a relation among lengths of the second bending portions is set to be contrary to a size relation among the outer diameters of the flexible tube portions in the insertion portions.

According to the aspect of the present invention, the endoscope system is realized in which, among models of the endoscopes including the insertion portions having the different outer diameters, the insertion portions suitable for insertion operation into a large intestine by the respective models are provided to obtain optimum insertability.

### Brief Description of the Drawings

Fig. 1 is an overall configuration diagram of an endoscope according to a first embodiment;
Fig. 2 is a diagram showing a configuration of an insertion portion according to the first embodiment;
Fig. 3 is a sectional view showing the configuration of the insertion portion according to the first embodiment;
Fig. 4 is a sectional view showing a configuration of an insertion portion of a modification of the first embodiment;
Fig. 5 is a diagram showing a state in which a small-diameter insertion portion is inserted into a large intestine in the first embodiment;
Fig. 6 is a diagram showing a state in which the small-diameter insertion portion is inserted into a large intestine deep part in the first embodiment;
Fig. 7 shows a state in which a large-diameter insertion portion is inserted up to a splenic flexure in the first embodiment;
Fig. 8 is a diagram showing a state in which the large-diameter insertion portion is subjected to twisting operation and traction operation to reduce in length and straighten an intestine in the first embodiment;
Fig. 9 is a diagram showing a state in which the large-diameter insertion portion is inserted up to a vicinity of a hepatic flexure in the first embodiment;
Fig. 10 is a diagram showing a state in which the large-diameter insertion portion is inserted into the large intestine deep part in the first embodiment;
Fig. 11 is a diagram showing a state in which a distal end of the insertion portion reaches the splenic flexure in the first embodiment;
Fig. 12 is a diagram showing a state in which the small-diameter insertion portion passes the splenic flexure in the first embodiment;
Fig. 13 is diagram showing a state in which the large-diameter insertion portion is hard to be hooked on the splenic flexure by a second bending portion in the first embodiment;
Fig. 14 is a diagram showing configurations of insertion portions having different diameters according to the first embodiment, wherein Fig. 14(a) is a diagram showing a configuration of an insertion portion of a smallest-diameter endoscope, Fig. 14(b) is a diagram showing a configuration of an insertion portion of an endoscope having a diameter larger than the diameter of the endoscope shown in Fig. 14(a), and Fig. 14(c) is a diagram showing a configuration of an insertion portion of a largest-diameter endoscope;
Fig. 15 is a diagram showing configurations of insertion portions having different diameters according to a second embodiment, wherein Fig. 15(a) is a diagram showing a configuration of an insertion portion of a smallest-diameter endoscope, Fig. 15(b) is a diagram showing a configuration of an insertion portion of an endoscope having a diameter larger than the diameter of the endoscope shown in Fig. 15(a), and Fig. 15(c) is a diagram showing a configuration of an insertion portion of a largest-diameter endoscope; and
Fig. 16 is a diagram showing configurations of insertion portions having different diameters according to a third embodiment, wherein Fig. 16(a) is a diagram showing a configuration of an insertion portion of a smallest-diameter endoscope, Fig. 16(b) is a diagram showing a configuration of an insertion portion of an endoscope having a diameter larger than the diameter of the endoscope shown in Fig. 15(a), and Fig. 16(c) is a diagram showing a configuration of an insertion portion of a largest-diameter endoscope.

### Best Mode for Carrying Out the Invention

An endoscope apparatus according to the present invention is explained below. Note that, in the following explanation, it should be noted that drawings based on respective embodiments are schematic and relations between thicknesses and widths of respective portions, ratios of thicknesses of the respective portions, and the like are different from actual ones. Among the drawings, portions having different relations and ratios of dimensions thereof are sometimes included.

Note that an endoscope in the following configuration example is explained with reference to, as an example, a so-called flexible endoscope in which an insertion portion to be inserted into a digestive organ in an upper part or a lower part of a living organism has flexibility. However, the endoscope is not limited to this and is a technique also applicable to a so-called rigid endoscope used for a surgical purpose in which an insertion portion is rigid.

### (First Embodiment)

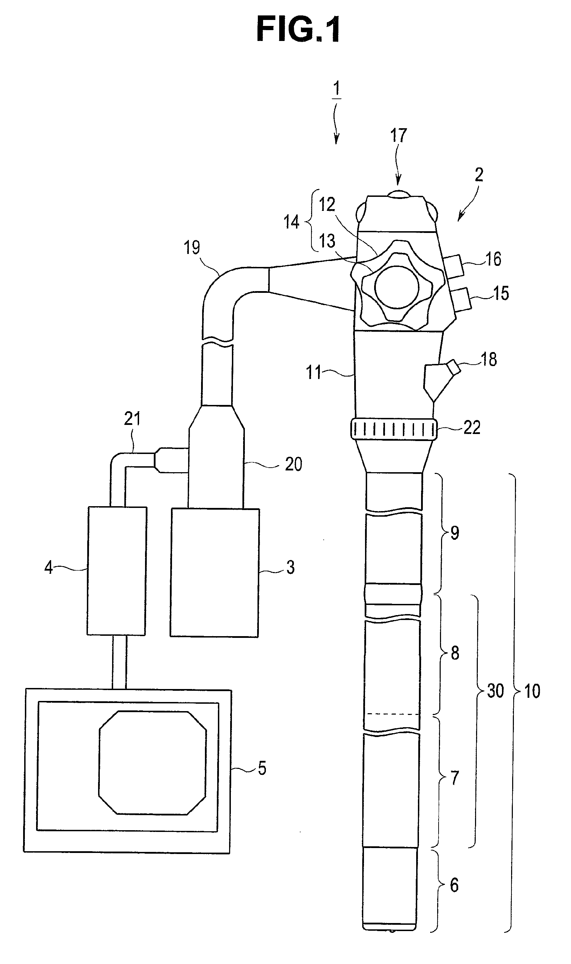

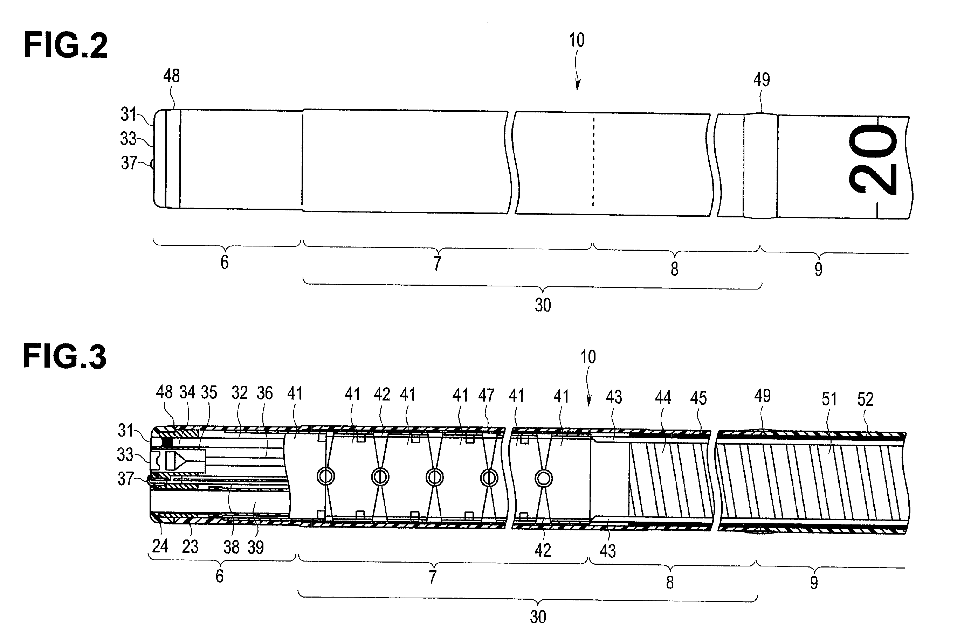

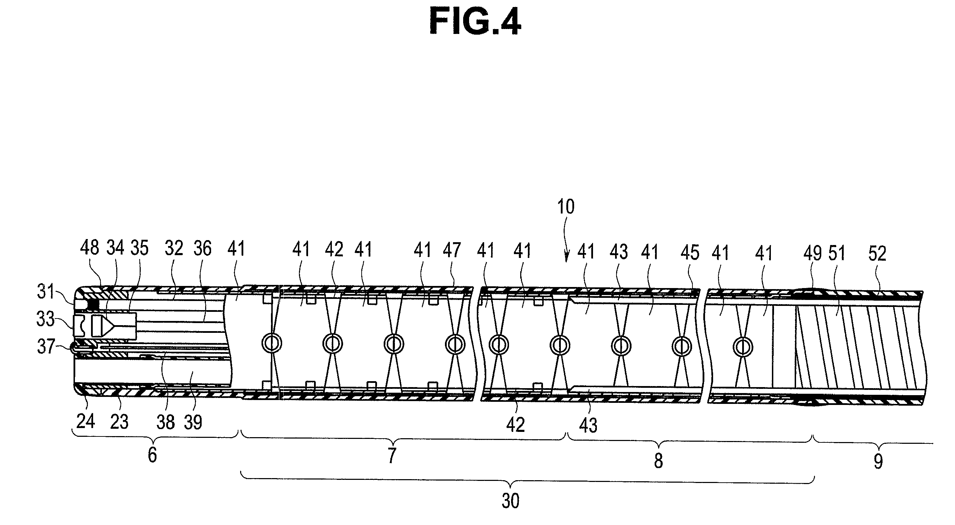

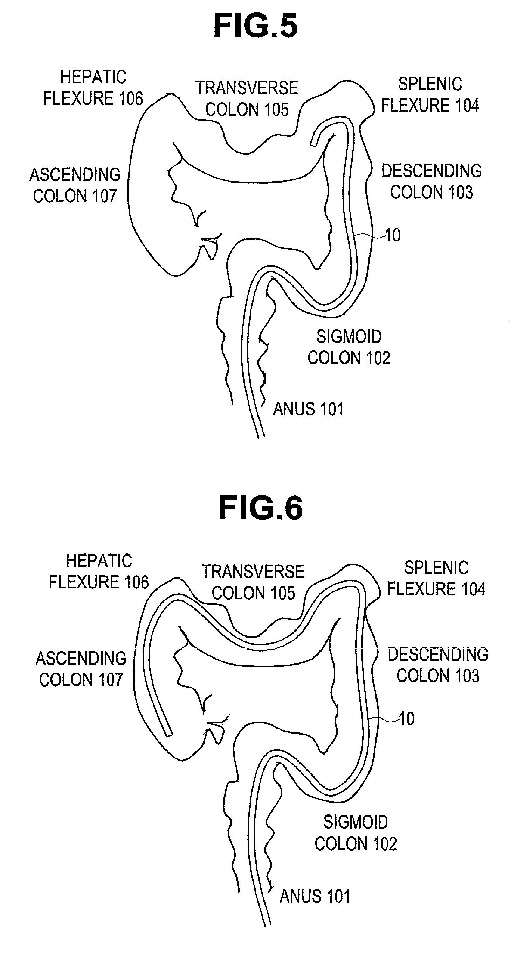

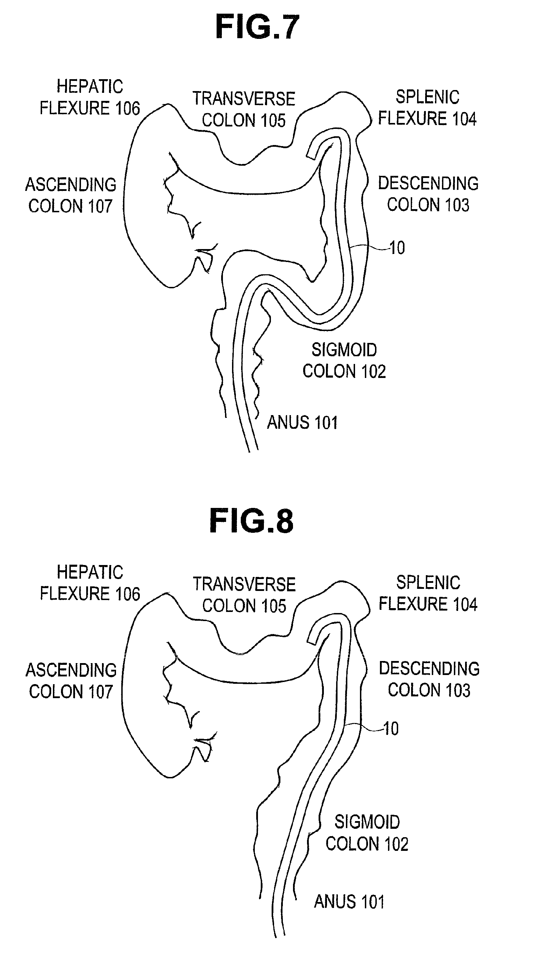

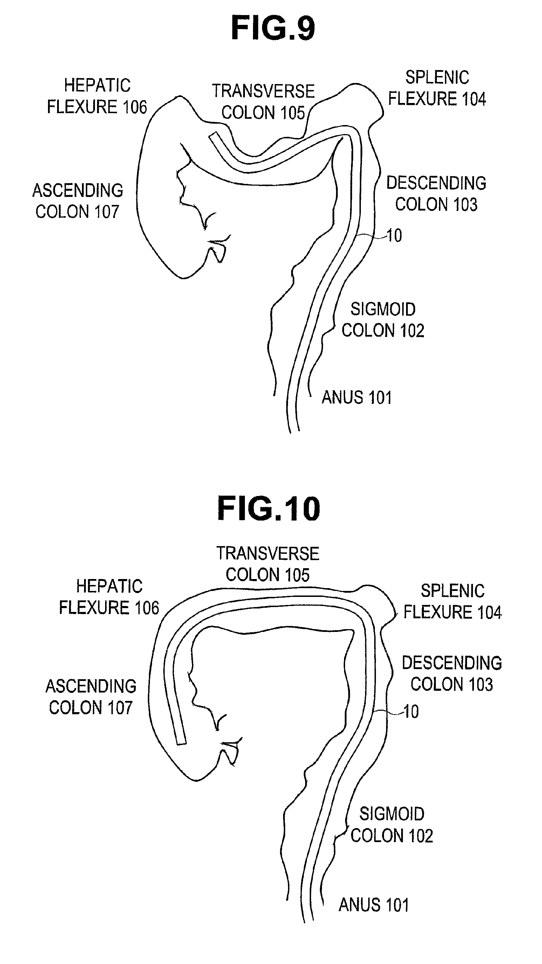

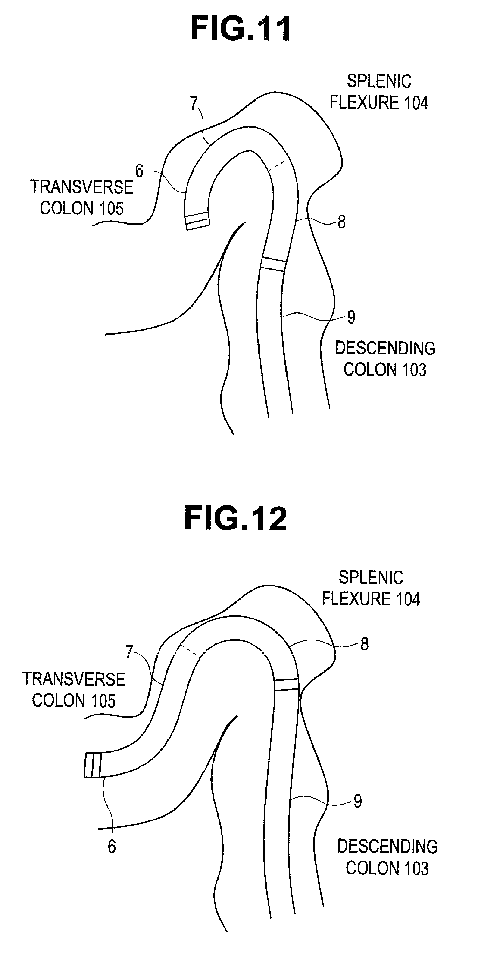

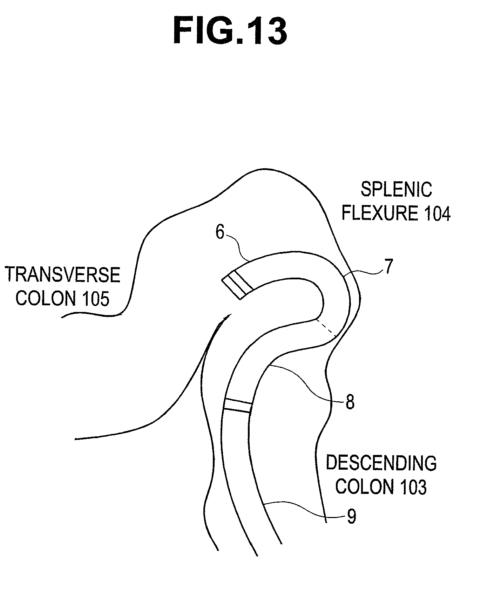

First, a first embodiment of the present invention is explained on the basis of the drawings. Figs. 1 to 14 relate to the first embodiment of the present invention. Fig. 1 is an overall configuration diagram of an endoscope according to the first embodiment. Fig. 2 is a diagram showing a configuration of an insertion portion. Fig. 3 is a sectional view showing the configuration of the insertion portion. Fig. 4 is a sectional view showing a configuration of an insertion portion of a modification. Fig. 5 is a diagram showing a state in which a small-diameter insertion portion is inserted into a large intestine. Fig. 6 is a diagram showing a state in which the small-diameter insertion portion is inserted into a large intestine deep part. Fig. 7 shows a state in which a large-diameter insertion portion is inserted up to a splenic flexure. Fig. 8 is a diagram showing a state in which the large-diameter insertion portion is subjected to twisting operation and traction operation to reduce in length and straighten an intestine. Fig. 9 is a diagram showing a state in which the large-diameter insertion portion is inserted up to a vicinity of a hepatic flexure. Fig. 10 is a diagram showing a state in which the large-diameter insertion portion is inserted into the large intestine deep part. Fig. 11 is a diagram showing a state in which a distal end of the insertion portion reaches the splenic flexure. Fig. 12 is a diagram showing a state in which the small-diameter insertion portion passes the splenic flexure. Fig. 13 is diagram showing a state in which the large-diameter insertion portion is hard to be hooked on the splenic flexure by a second bending portion.

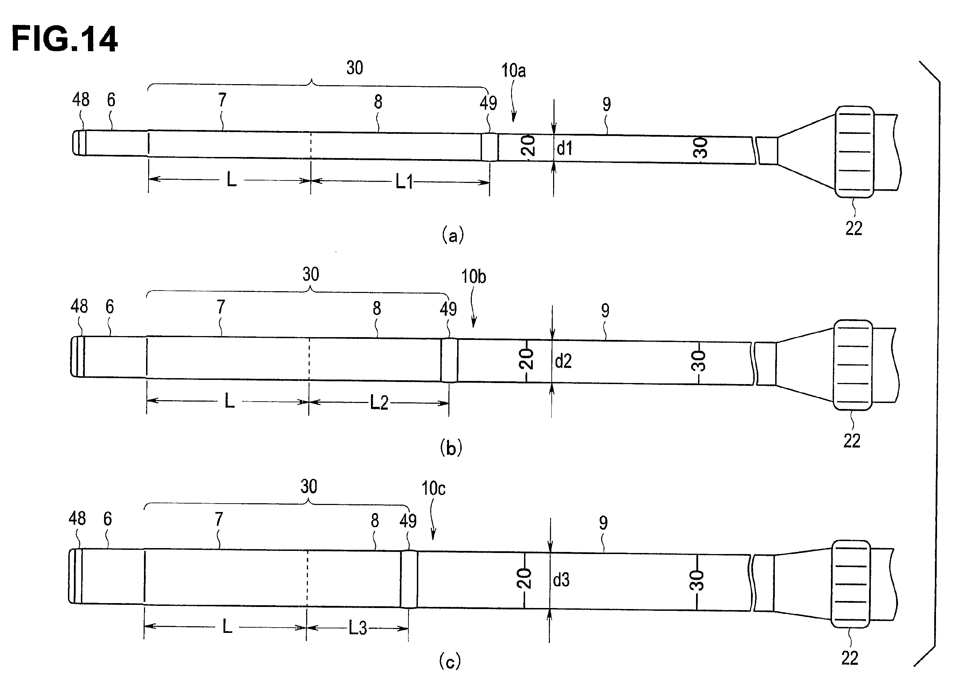

Fig. 14 is a diagram showing configurations of insertion portions having different diameters. Fig. 14(a) is a diagram showing a configuration of an insertion portion of a smallest-diameter endoscope, Fig. 14(b) is a diagram showing a configuration of an insertion portion of an endoscope having a diameter larger than the diameter of the endoscope shown in Fig. 14(a), and Fig. 14(c) is a diagram showing a configuration of an insertion portion of a largest-diameter endoscope.

As shown in Fig. 1, an endoscope apparatus 1 according to the present embodiment is mainly configured with an endoscope 2, a light source device 3, a video processor 4, and a monitor 5.

The endoscope 2 includes a long and elongated insertion portion 10, an operation portion 11, and a universal cable 19. The insertion portion 10 of the endoscope 2 includes, in order from a distal end side, a distal end portion 6, a bending portion 30, and a flexible tube portion 9. Further, the bending portion 30 is configured by, in order from the distal end side, a first bending portion 7 and a second bending portion 8. Note that a detailed configuration of the insertion portion 10 is explained in detail below.

The operation portion 11, from which the flexible tube portion 9 of the insertion portion 10 is extended from a bend preventing portion, includes a treatment instrument channel insertion portion 18, which is an opening portion for a below-mentioned treatment instrument channel 39 (see Fig. 3) and through which various treatment instruments disposed in the insertion portion 10 are inserted.

In the operation portion 11, a bending operation knob 14 for subjecting the first bending portion 7 of the insertion portion 10 to bending operation is turnably disposed and switches 17 and the like for various endoscope functions are provided. Note that the bending operation knob 14 is disposed such that a UD bending operation knob 12 for subjecting the first bending portion 7 to the bending operation in an up down direction and an RL bending operation knob 13 for subjecting the first bending portion 7 to the bending operation in a left right direction are superimposed. In the operation portion 11, a hardness variable dial 22 capable of changing hardness of the flexible tube portion 9 of the insertion portion 10 is provided.

The universal cable 19 extended from the operation portion 11 includes, at an extension end, an endoscope connector 20 detachably attachable to the light source device 3. Note that the endoscope 2 according to the present embodiment transmits illumination light from the light source device 3 to the distal end portion 6 using the universal cable 19, the operation portion 11, and a light guide bundle 32 (see Fig. 3) of below-mentioned illuminating means disposed in the insertion portion 10. A detachably attachable cable 21 is extended from the endoscope connector 20. An extension end of the cable 21 is configured to be detachably attachable to the video processor 4 by an electric connector.

The video processor 4 is electrically connected to the monitor 5 that displays an endoscopic image. The video processor 4 subjects an image pickup signal photoelectrically converted by a below-mentioned image pickup unit for an endoscope, which is image pickup means, of the endoscope 2 to signal processing and outputs the image pickup signal to the monitor 5 as an image signal. Note that, in the endoscope apparatus 1, although not shown in the figure, an air/water feeding function for jetting air and water from the distal end portion 6 of the insertion portion 10 of the endoscope 2 is provided in the light source device 3.

Next, a configuration of the insertion portion 10 of the endoscope 2 is explained below on the basis of Figs. 2 and 3.

The insertion portion 10 in the present embodiment includes, as shown in Figs. 2 and 3, the rigid distal end portion 6, the bending portion 30 including the first bending portion 7 actively subjected to bending operation and a extremely flexible second bending portion 8 to be passively bent, and the flexible tube portion 9, hardness of which is set higher than hardness of the second bending portion 8. Note that the second bending portion 8 functions as a passive type bending portion that cannot be subjected to the bending operation by the operation portion 14 but receives external force to be bent.

In the distal end portion 6, as shown in Fig. 3, a distal end cover 24 made of resin is disposed in a distal end rigid portion 23, which is a metal block. In the distal end portion 6, an illumination optical system 31 functioning as an illumination window is disposed to a distal end face on which the distal end cover 24 is provided. An end portion of the light guide bundle 32 configured to transmit illumination light from the light source device 3 is arranged behind the illumination optical system 31. That is, the illumination light transmitted by the light guide bundle 32 is emitted forward from the illumination optical system 31 disposed on the distal end face of the distal end portion 6 and illuminates an object such as a diseased part.

In the distal end portion 6, an image pickup unit 35 including an objective lens 33, which is an observation optical system functioning as an observation window, and a solid-state image pickup device (CCD, CMOS, etc.) 34 functioning as a charge coupled device provided in an image-forming of the objective lens 33 and including a function of photoelectrically converting an optical image is provided. Note that a communication cable 36 is extended from the image pickup unit 35.

An air/water feeding nozzle 37 configured to feed air and feed water to a surface of the objective lens 33 is provided on the distal end face of the distal end portion 6. One end of an air/water feeding conduit 38 is connected to the air/water feeding nozzle 37. Further, an opening of the treatment instrument channel 39 is provided on the distal end face of the distal end portion 6.

The first bending portion 7, which is a part of the bending portion 30 provided adjacent to the distal end portion 6, is configured by turnably coupling a large number of ring-shaped bending pieces 41 to one another by rivets or the like in positions corresponding to above and below and left and right of the bending pieces 41 adjacent to one another. Note that an end portion of a bending wire 42 is fixed to the bending piece 41 at a most distal end. The bending wire 42 is inserted through a coil sheath 43 disposed up to a distal end of the second bending portion 8 in the insertion portion 10. A rear end of the bending wire 42 is coupled to a not-shown sprocket in the operation portion 11.

The sprocket is coupled to the bending operation knob 14. By performing operation for turning the UD bending operation knob 12 or the RL bending operation knob 13, one of a pair of bending wires 42 arranged along the up down direction or the left right direction is pulled and the other is loosened to make it possible to bend the first bending portion 7 to the pulled bending wire 42 side. That is, the first bending portion 7 configures an active type flexible portion that actively bends according to operation of the bending operation knob 14.

In the second bending portion 8, which is a part of the bending portion 30, a spiral tube (also referred to as flex tube) 44 and a reticulated tube (also referred to as braid) 45 configured to cover the spiral tube 44 are provided. In the second bending portion 8, an outer circumference of the reticulated tube 45 is covered by flexible bending rubber 47 functioning as a skin. Note that the bending rubber 47 is connected to the distal end cover 24 of the distal end portion 6 and integrally covers the bending portion 30 from behind the distal end portion 6. A distal end portion of the bending rubber 47 is fixed by a thread wound bonding portion 48.

The second bending portion 8 is set to have predetermined hardness (rigidity) according to rigidity of the reticulated tube 45 and the bending rubber 47 that cover the spiral tube 44. Note that the predetermined hardness of the second bending portion 8 is set lower than predetermined hardness of the flexible tube portion 9 explained below. The second bending portion 8 is configured to be extremely flexible. The second bending portion 8 configures a passive type flexible portion to be passively bent by external force.

In the flexible tube portion 9, as in the second bending portion 8, a spiral tube 51 and a reticulated tube 52 configured to cover the spiral tube 51 are provided. In the flexible tube portion 9, an outer circumference of the reticulated tube 45 is covered by a resin tube 53 functioning as a skin. Note that a proximal end portion of the bending rubber 47 and a distal end portion of the resin tube 53 are fixed by a thread wound bonding portion 49.

The resin tube 53 is applied with drug resistant coating on a surface of an outer circumference. Hardness of the resin tube 53 is set such that the flexible tube portion 9 has predetermined hardness (rigidity). That is, as explained above, the predetermined hardness of the flexible tube portion 9 is set higher than the predetermined hardness of the second bending portion 8 according to rigidity of the resin tube 53. The flexible tube portion 9 is configured to have predetermined flexibility (so-called resilience) necessary for pushing operation into a body cavity (here, a large intestine). Note that, here, the spiral tube 51 and the reticulated tube 52 of the flexible tube portion 9 are formed in an integral configuration continuous to the spiral tube 44 and the reticulated tube 45 of the second bending portion 8. Note that the spiral tubes 44 and 51 and the reticulated tubes 45 and 52 may be provided as separate bodies in the second bending portion 8 and the flexible tube portion 9.

In the present embodiment, a difference in hardness (rigidity) between the second bending portion 8 and the flexible tube portion 9 is set according to a difference in hardness (rigidity) between the bending rubber 47 and the resin tube 53. As explained above, the second bending portion 8 is set extremely soft. The hardness of the flexible tube portion 9 is set to be harder than the second bending portion 8 and, in particular, to have predetermined flexibility necessary for insertion into a large intestine.

Note that the hardness of the second bending portion 8 and the flexible tube portion 9 may be set by changing a spiral pitch, thickness, or the like of the spiral tubes 44 and 51 in addition to the difference in hardness between the bending rubber 47 and the resin tube 53. Further, as shown in Fig. 4, a plurality of bending pieces 41 may be provided as in the first bending portion 7 instead of the spiral tube 44 and the reticulated tube 45 to configure the second bending portion 8 to be passively bent.

The insertion portion 10 configured as explained above has a configuration in which the first bending portion 7, the second bending portion 8, and the flexible tube portion 9 have substantially the same diameters (outer diameters).

Here, insertion operation for inserting the insertion portion 10 of the endoscope 2 configured as explained above into a large intestine of a patient according to thickness of the insertion portion 10 is explained.

First, in the endoscope 2 including the small-diameter insertion portion 10, as shown in Fig. 5, the insertion portion 10 is inserted from an anus 101 and pushed in simultaneously with bending operation of the first bending portion 7. Then, a distal end of the insertion portion 10 easily reaches a winding sigmoid colon 102, a descending colon 103, and a splenic flexure 104. In the endoscope 2 including the small-diameter insertion portion 10, the first bending portion 7 is subjected to bending operation according to a bent shape of the splenic flexure 104 and the distal end of the insertion portion 10 is directed to a transverse colon 105 and subjected to push-in operation. Consequently, as shown in Fig. 5, the distal end of the insertion portion 10 can pass through the splenic flexure 104.

Further, in the endoscope 2 including the small-diameter insertion portion 10, when the insertion portion 10 passes through a hepatic flexure 106, similarly, the first bending portion 7 is subjected to bending operation according to a bent shape of the hepatic flexure 106 and the distal end of the insertion portion 10 is directed to an ascending colon 107 side and simply subjected to push-in operation. Then, the insertion portion 10 can easily pass through the hepatic flexure 106. In this way, in the endoscope 2 including the small-diameter insertion portion 10, since an outer diameter of the insertion portion 10 is small, the entire insertion portion 10 is relatively flexible compared with a large-diameter insertion portion. Therefore, in insertion operation up to a large intestine deep part shown in Fig. 6, simple push-in operation is often performed.

On the other hand, in the endoscope 2 including the large-diameter insertion portion 10, as shown in Fig. 7, the insertion portion 10 is inserted from the anus 101 and pushed in simultaneously with bending operation of the first bending portion 7. Then, the distal end of the insertion portion 10 reaches the winding sigmoid colon 102, the descending colon 103, and the splenic flexure 104. In the endoscope 2 including the large-diameter insertion portion 10, the first bending portion 7 is subjected to the bending operation and the distal end of the insertion portion 10 is hooked on a bend of the splenic flexure 104 and subjected to twisting operation and traction operation. According to the twisting operation and the traction operation, as shown in Fig. 8, the large-diameter insertion portion 10 is subjected to push-in operation in a state in which the anus 101 to the sigmoid colon 102 and the descending colon 103 are reduced in length and straightened. Consequently, as shown in Fig. 9, the distal end of the insertion portion 10 can pass through the splenic flexure 104.

Further, in the large-diameter insertion portion 10, since the distal end of the insertion portion 10 is hooked and subjected to the traction operation, the distal end of the insertion portion 10 is hooked on the bend of the hepatic flexure 106. As shown in Fig. 10, the transverse colon 105 is changed to a lifted state by the twisting operation and the traction operation. The distal end of the insertion portion 10 passes the hepatic flexure 106 and is inserted up to a large intestine deep part. In this way, in the endoscope 2 including the large-diameter insertion portion 10, since the outer diameter of the insertion portion 10 is large, the entire insertion portion 10 has the predetermined rigidity compared with a small-diameter insertion portion. Therefore, in inserting the distal end of the insertion portion 10 up to the large intestine deep part shown in Fig. 10, operation for pushing in the distal end of the insertion portion 10 after reducing in length and straightening the intestine with the twisting operation and the traction operation is necessary.

Note that, as explained above, in the small-diameter insertion portion 10, as shown in Figs. 11 and 12, when it is attempted to allow the distal end of the insertion portion 10 to pass, for example, the bend of the splenic flexure 104 only with the push-in operation, the flexible second bending portion 8 is passively bent along the bent shape. The second bending portion 8 is prevented from pushing up an intestinal wall of the bend and hindering an advance of the distal end of the insertion portion 10. The distal end of the insertion portion 10 can smoothly pass through the splenic flexure 104. However, in the large-diameter insertion portion 10, as shown in Fig. 13, for example, when it is attempted to subject the distal end of the insertion portion 10 to the twisting operation and the traction operation to straighten the intestine in a state in which the distal end of the insertion portion 10 is hooked on the bend of the splenic flexure 104, resilience is lost because the second bending portion 8 is flexible. In some case, the distal end of the insertion portion 10 is not successfully hooked on the bend of the splenic flexure 104 and comes off. Consequently, it may be difficult for the large-diameter insertion portion 10 including the second bending portion 8 to straighten the intestine.

Therefore, in the insertion portion 10 of the endoscope 2 in the present embodiment, a length in a longitudinal (insertion portion axis) direction of the second bending portion 8 is set larger as a diameter of the insertion portion 10 is smaller. The length in the longitudinal direction (insertion portion axis) direction of the second bending portion 8 is set smaller as the diameter of the insertion portion 10 is larger. More specifically, as shown in Fig. 14, here, three insertion portions 10 of the endoscopes 2 having different diameters d1, d2, and d3 are illustrated and explained in detail. Note that Fig. 14(a) is a diagram showing a configuration of an insertion portion of a smallest-diameter endoscope, Fig. 14(b) is a diagram showing a configuration of an insertion portion of an endoscope having a diameter larger than the diameter of the endoscope shown in Fig. 14(a), and Fig. 14(c) is a diagram showing a configuration of an insertion portion of a largest-diameter endoscope.

Note that, in an insertion portion 10a (the flexible tube portion 9) shown in Fig. 14(a), the diameter (an outer diameter) d1 is set to, for example, 9 mm (d1=9mm). In an insertion portion 10b (the flexible tube portion 9) shown in Fig. 14(b), the diameter (an outer diameter) d2 is set to, for example, 11 mm (d1=11mm). In an insertion portion 10c (the flexible tube portion 9) shown in Fig. 14(c), the diameter (an outer diameter) d3 is set to, for example, 13 mm (d3=13mm). In this way, the three endoscopes 2 shown in Fig. 14 respectively include the insertion portions 10a, 10b, and 10c (the flexible tube portions 9) respectively having the different diameters d1, d2, and d3. A relation among thicknesses (thinnesses) of the insertion portions 10a, 10b, and 10c is diameter d1<diameter d2<diameter d3. Here, the three insertion portions 10a, 10b, and 10c of the endoscopes 2 include the first bending portions 7 having substantially the same lengths L in a longitudinal direction.

In the insertion portion 10a in which at least the flexible tube portion 9 is set to the smallest (shortest) diameter d1, a length L1 in the longitudinal direction of the second bending portion 8 is set. In the insertion portion 10b in which at least the flexible tube portion 9 is set to the intermediate diameter d2, a length L2 in the longitudinal direction of the second bending portion 8 is set. In the insertion portion 10a in which at least the flexible tube portion 9 is set to the largest (longest) diameter d3, a length L3 in the longitudinal direction of the second bending portion 8 is set.

Therefore, in the three insertion portions 10a, 10b, and 10c, as a relation among the lengths of the respective second bending portions 8, the length L1 of the second bending portion 8 of the insertion portion 10a is set largest, the length L2 of the second bending portion 8 of the insertion portion 10b is set second largest, and the length L3 of the second bending portion 8 of the insertion portion 10c is set smallest (L1>L2>L3). That is, in the insertion portion 10, a length of the second bending portion 8 is set larger as at least the flexible tube portion 9 has a smaller diameter. The length of the second bending portion 8 is set smaller as at least the flexible tube portion 9 has a larger diameter.

Note that, here, with reference to the three insertion portions 10a, 10b, and 10c as an example, the length relation (L1>L2>L3) of the lengths L1, L2, and L3 of the respective second bending portions 8 is explained. However, the insertion portions 10 are not limited to these three insertion portions 10a, 10b, and 10c. Among models of the plurality of endoscopes 2, the length of the second bending portion 8 is set to be larger in order from the insertion portion 10 having a smallest length and is set to be smaller in order from the insertion portion 10 having a largest length. That is, in the plurality of insertion portions 10, a length relation among the second bending portions 8 is set contrary to a size relation among the diameters of the insertion portions 10.

In this way, in the endoscope system including the plurality of endoscopes 2, during insertion into the large intestine, which is the body cavity, rates of the push-in operation and the reduction in length and straightening of the intestine by the twisting operation and the traction operation change according to the diameters of the insertion portions 10 among the respective models. Therefore, the length of the second bending portion 8, which is passively bent by external force, is changed and set according to these kinds of insertion operation. That is, in the small-diameter insertion portion 10, since a rate of insertion into the large intestine deep part by the push-in operation is large, a length of the flexible second bending portion 8 is set larger than a length of the large-diameter insertion portion 10 such that, when the insertion portion 10 passes respective bends of the large intestine, the insertion portion 10 can smoothly pass through the respective bends without pushing up the intestinal wall. On the other hand, in the large-diameter insertion portion 10, a rate of subjecting the distal end of the insertion portion 10 to the twisting operation and the traction operation, reducing in length and straightening the intestine, and inserting the distal end of the insertion portion 10 in a state in which the distal end of the insertion portion 10 is hooked on the respective bends of the large intestine is large. Therefore, the length of the flexible second bending portion 8 is set smaller than the length of the small-diameter insertion portion 10 such that the distal end of the insertion portion 10 does not come off and the intestine is easily reduced in length and straightened.

As explained above, the endoscope system including the plurality of endoscopes 2 is configured such that, among the models of the plurality of endoscopes 2 including the insertion portions 10 in which the outer diameters of at least the flexible tube portions 9 are different, the length in the longitudinal direction of the second bending portion 8 is set contrary to the diameter of the different insertion portion 10 to match the insertion operation into the large intestine corresponding to the diameters of the respective insertion portions 10. Therefore, optimum insertability is obtained.

### (Second Embodiment)

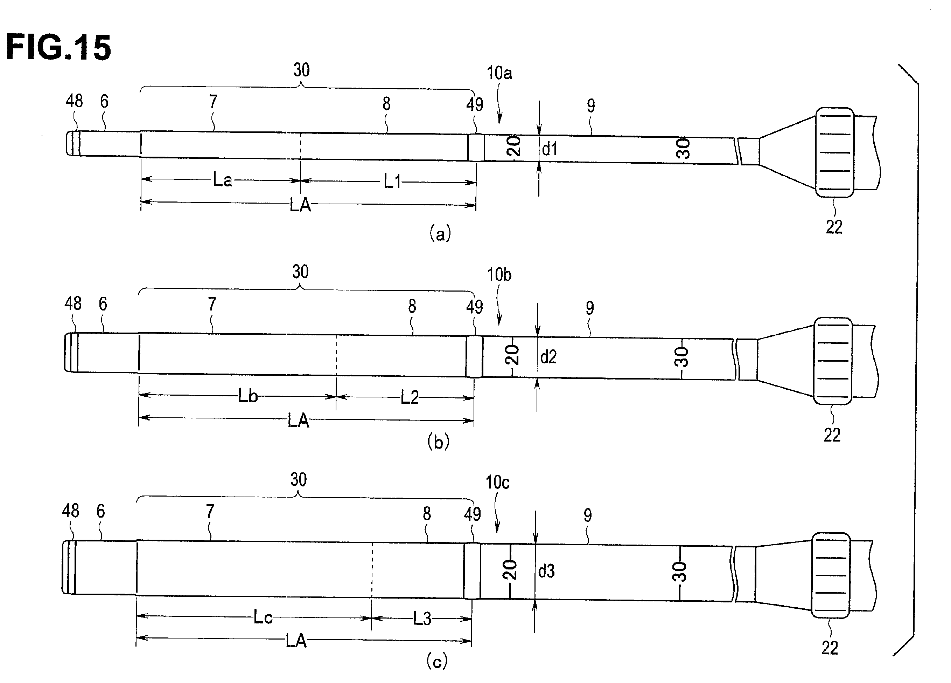

Next, a second embodiment is explained on the basis of Fig. 15. The present embodiment is a modification of the first embodiment. The same members are denoted by the same reference numerals and signs and detailed explanation of the members is omitted. Fig. 15 relates to the second embodiment of the present invention and is a diagram showing configurations of insertion portions having different diameters. Fig. 15(a) is a diagram showing a configuration of an insertion portion of a smallest-diameter endoscope, Fig. 15(b) is a diagram showing a configuration of an insertion portion of an endoscope having a diameter larger than the diameter of the endoscope shown in Fig. 15(a), and Fig. 15(c) is a diagram showing a configuration of an insertion portion of a largest-diameter endoscope.

In the insertion portion 10 of the endoscope 2 in the present embodiment, as in the first embodiment, the length of the second bending portion 8 is set larger as the diameter of the endoscope 2 is smaller. The length of the second bending portion 8 is set smaller as the diameter of the endoscope 2 is larger. More specifically, as shown in Fig. 15, here, as in the first embodiment, the three insertion portions 10 of the endoscopes 2 having different diameters d1, d2, and d3 are illustrated and explained in detail.

Note that, as in the first embodiment, the diameter (the outer diameter) d1 of the insertion portion 10a (the flexible tube portion 9) shown in Fig. 15(a) is set to, for example, 9 mm (d1=9mm), the diameter (the outer diameter) d2 of the insertion portion 10b (the flexible tube portion 9) shown in Fig. 15(b) is set to, for example, 11 mm (d1=11mm), and the diameter (the outer diameter) d3 of the insertion portion 10c (the flexible tube portion 9) shown in Fig. 15(c) is set to, for example, 13 mm (d3=13mm). Here, as in the first embodiment, in the three endoscopes 2 shown in Fig. 15, a relation among the thicknesses (the thinnesses) of the respective insertion portions 10a, 10b, and 10c is diameter d1<diameter d2<diameter d3.

Here, the insertion portions 10a, 10b, and 10c of the three endoscopes 2 include the bending portions 30 for which substantially the same lengths LA in the longitudinal direction are set. That is, in the three insertion portions 10a, 10b, and 10c, sums of lengths La, Lb, and Lc in the longitudinal direction of the respective first bending portions 7 and lengths L1, L2, and L3 in the longitudinal direction of the respective second bending portions 8 are set to substantially same lengths LA (=La+L1=Lb+L2=Lc+L3).

Here, as in the first embodiment, in the three insertion portions 10a, 10b, and 10, as a relation among the lengths of the respective second bending portions 8, the length L1 of the second bending portion 8 of the insertion portion 10a is set largest, the length L2 of the second bending portion 8 of the insertion portion 10b is set second largest, and the length L3 of the second bending portion 8 of the insertion portion 10c is set smallest (L1>L2>L3).

In the insertion portions 10a, 10b, and 10c of the three endoscopes 2, since the lengths LA of the bending portions 30 are the same, as a relation among the lengths of the respective first bending portions 7, the length La of the first bending portion 7 of the insertion portion 10a is set smallest, the length Lb of the first bending portion 7 of the insertion portion 10b is set second smallest, and the length Lc of the first bending portion 7 of the insertion portion 10c is set largest (La<Lb<Lc). That is, in the insertion portion 10, the length of the first bending portion 7 is set smaller as the diameter of at least the flexible tube portion 9 is smaller. The length of the first bending portion 7 is set larger as the diameter of the flexible tube portion 9 is larger.

The three endoscopes 2 include the bending portions 30 having substantially the same lengths. Respective ratios of the lengths La, Lb, Lc of the first bending portions 7 and the length L1, L2, and L3 of the second bending portions 8 are different with respect to the diameters d1, d2, and d3 of at least the flexible tube portions 9 of the insertion portions 10.

Note that, here, as in the first embodiment, the three insertion portions 10a, 10b, and 10c are explained as an example. However, the insertion portions 10 are not limited to these three insertion portions 10a, 10b, and 10c. Among models of the plurality of endoscopes 2, the length of the second bending portion 8 is set to be larger in order from the insertion portion 10 having a smallest diameter and is set to be smaller in order from the insertion portion 10 having a largest diameter.

Even in the configuration explained above, in the endoscope system including the plurality of endoscopes 2 in the present embodiment, a length relation among the second bending portions 8 is set contrary to a size relation among the diameters of at least the flexible tube portions 9 of the insertion portions 10. Therefore, the action and effects described in the first embodiment are attained. In addition, the length of the first bending portion 7 is larger as a rate of hooking the distal end of the insertion portion 10 on respective bends of a large intestine and subjecting the distal end of the insertion portion 10 to twisting operation and traction operation is higher.
Therefore, there is an advantage that the distal end of the insertion portion 10 is easily hooked on the bends and less easily comes off. Further, in the endoscopes 2, the lengths LA of the bending portions 30 configured by the first bending portions 7 and the second bending portions 8 are the same among the models, regions from the distal end portions 6 to the second bending portions 8 set as proximal ends of the bending portions 30 are unified. Therefore, distance senses of insertion into the large intestine become the same and the endoscope system is configured to be capable of being used without a sense of discomfort.

### (Third Embodiment)

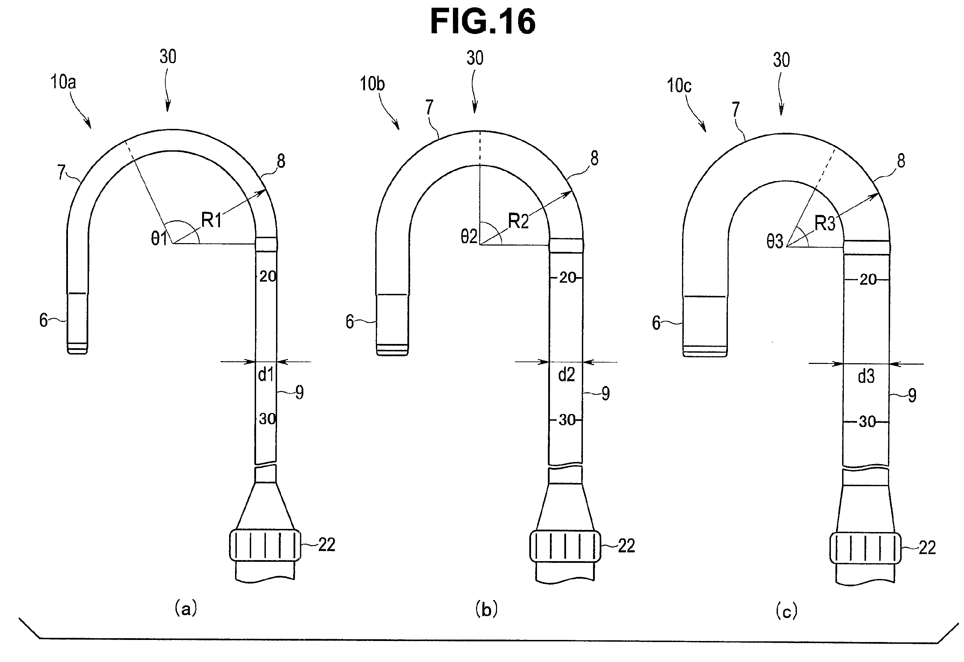

Next, a third embodiment is explained on the basis of Fig. 16. The present embodiment is also a modification of the first embodiment. The same members are denoted by the same reference numerals and signs and detailed explanation of the members is omitted. Note that a configuration of the present embodiment can be applied to the configuration of the second embodiment as well. Fig. 16 relates to the third embodiment of the present invention and is a diagram showing configurations of insertion portions having different diameters. Fig. 16(a) is a diagram showing a configuration of an insertion portion of a smallest-diameter endoscope, Fig. 16(b) is a diagram showing a configuration of an insertion portion of an endoscope having a diameter larger than the diameter of the endoscope shown in Fig. 16(a), and Fig. 16(c) is a diagram showing a configuration of an insertion portion of a largest-diameter endoscope.

In the present embodiment, as in the first embodiment, the diameter (the outer diameter) d1 of the insertion portion 10a (the flexible tube portion 9) shown in Fig. 16(a) is set to, for example, 9 mm (d1=9mm), the diameter (the outer diameter) d2 of the insertion portion 10b (the flexible tube portion 9) shown in Fig. 16(b) is set to, for example, 11 mm (d1=11mm), and the diameter (the outer diameter) d3 of the insertion portion 10c (the flexible tube portion 9) shown in Fig. 16(c) is set to, for example, 13 mm (d3=13mm). Here, as in the first embodiment, in the three endoscopes 2 shown in Fig. 15, a relation among the thicknesses (the thinnesses) of the respective insertion portions 10a, 10b, and 10c is diameter d1<diameter d2<diameter d3.

Here, in the insertion portions 10a, 10b, and 10c of the three endoscopes 2, respective curvature radiuses R1, R2, and R3 on the outside in a state in which the respective second bending portions 8 are bent to the maximum are set to be substantially the same (R1=R2=R3). That is, in the respective second bending portions 8 of the insertion portions 10a, 10b, and 10c, arcs on the outside in a state in which the second bending portions 8 are bent to the maximum are set to have substantially the same curvatures (1/R1=1/R2=1/R3).

The respective second bending portions 8 here are provided with the plurality of bending pieces 41 shown in Fig. 4. The curvature radiuses R1, R2, and R3 of arcs drawn by respective outer peripheral portions on outer sides, that is, the curvatures (1/R1, 1/R2, and 1/R3) in a state in which the second bending portions 8 are bent to the maximum are set to be substantially the same (1/R1=1/R2=1/R3) according to setting of respective piece dimensions.

In the three insertion portions 10a, 10b, and 10c in the present embodiment, as a relation among bending angels at the time when the respective second bending portions 8 are bent to the maximum, a maximum bending angle θ1 of the second bending portion 8 of the insertion portion 10a is set largest, a maximum bending angle θ2 of the second bending portion 8 of the insertion portion 10b is set second largest, and a maximum bending angle θ3 of the second bending portion 8 of the insertion portion 10c is set smallest (θ1>θ2>θ3). Note that, here, as in the first embodiment, the three insertion portions 10a, 10b, and 10c are explained as an example. However, the insertion portions 10 are not limited to these three insertion portions 10a, 10b, and 10c. Among models of the plurality of endoscopes 2, the maximum bending angle of the second bending portion 8 is set to be larger in order from the insertion portion 10 having a smallest diameter of at least the flexible tube portion 9 and is set to be smaller in order from the insertion portion 10 having a largest diameter of at least the flexible tube portion 9. That is, in the plurality of insertion portions 10, a relation among the maximum bending angles of the second bending portions 8 is set contrary to a size relation among the diameters of the insertion portions 10.

As a result, the length of the second bending portion 8 is set to be larger in order from the insertion portion 10 having a smallest diameter of at least the flexible tube portion 9 and is set to be smaller in order from the insertion portion 10 having a largest diameter of at least the flexible tube portion 9. That is, in the small-diameter insertion portion 10, the length of the second bending portion 8 is set larger than that of the large-diameter insertion portion 10 by setting the maximum bending angle of the second bending portion 8 large. In the large-diameter insertion portion 10, the length of the second bending portion 8 is set smaller than that of the small-diameter insertion portion 10 by setting the maximum bending angle of the second bending portion 8 small.

Even in the configuration explained above, in the endoscope system including the plurality of endoscopes 2 in the present embodiment, a relation among the maximum bending angles of the second bending portions 8 is set contrary to a size relation among the diameters of at least the flexible tube portions 9 of the insertion portions 10. According to this setting, a length relation among the second bending portions 8 is also contrary to the size relation among the diameters of the insertion portions 10. Therefore, the effects described in the first embodiment are attained. In addition, since curvature radiuses of arcs drawn by respective outer peripheral portions on outer sides, that is, curvatures in a state in which the second bending portions 8 are bent to the maximum are set to be substantially the same, bending shapes of maximum bends of the respective second bending portions 8 coming into contact with the intestinal wall of the large intestine and bent by external force are unified. Therefore, insertion senses of insertion of the insertion portions 10 into the large intestine become the same and the endoscope system is configured to be capable of being used without a sense of discomfort.

The invention described in the embodiments is not limited to the embodiments and the modifications thereof. Besides, in an implementation stage, various modifications can be carried out without departing from the scope of the claims.

Further, inventions in various stages are included in the embodiments. Various inventions can be extracted according to appropriate combinations in a plurality of constituent features disclosed herein.

For example, when the problems explained herein can be solved and the effects explained herein can be obtained even if several constituent features are deleted from all the constituent features explained in the embodiments, a configuration from which the constituent features are deleted can be extracted as an invention.

The present application is filed claiming the priority of Japanese Patent Application No. 2012-110802 filed in Japan on May 14, 2012.

## Claims

1. An endoscope system comprising a plurality of endoscopes including insertion portions configured by bending portions (7,8) and flexible tube portions (9), outer diameters of at least the flexible tubes being different from one another, wherein
each of the bending portions in the plurality of endoscopes includes:
a first bending portion (7) subjected to bending operation to actively bend; and
a second bending portion (8) connected to the first bending portion and passively bent by external force,
the flexible tube portions (9) are respectively connected to the second bending portion and formed to have rigidity higher than the rigidity of the second bending portion,
and
in the plurality of endoscopes, a relation among lengths (L1, L2, L3) of the second bending portions is set to be contrary to a size relation among the outer diameters (d1, d2, d3) of the flexible tube portions in the insertion portions.

2. The endoscope system according to claim 1, wherein lengths of the first bending portions of the plurality of endoscopes are substantially the same.

3. The endoscope system according to claim 1, wherein lengths of the entire bending portions including the first bending portions and the second bending portions of the plurality of endoscopes are substantially the same.

4. The endoscope system according to any one of claims 1 to 3, wherein, in the second bending portions, a size relation among maximum bending angles is set to be contrary to a size relation among diameters of the insertion portions.

5. The endoscope system according to any one of claims 1 to 4, wherein, in the second bending portions, arcs drawn by outer peripheral portions on outer sides during maximum bending are set to substantially same curvatures.

6. The endoscope system according to claim 1, wherein an outer diameter of the second bending portion is substantially equal to the outer diameter of the flexible tube portion.

7. The endoscope system according to claim 1, wherein an outer diameter of the first bending portion is substantially equal to the outer diameter of the flexible tube portion.

## Patentansprüche

1. Endoskopsystem, das eine Mehrzahl von Endoskopen aufweist, die Einführbereiche beinhalten, die durch Biegebereiche (7,8) und Schlauchbereiche (9) ausgebildet sind, wobei Außendurchmesser mindestens der Schläuche sich voneinander unterscheiden, wobei
die Biegebereiche bei der Mehrzahl von Endoskopen jeweils beinhalten:
einen ersten Biegebereich (7), der einem Biegevorgang so unterzogen wird, dass er sich aktiv biegt; und
einen zweiten Biegebereich (8), der mit dem ersten Biegebereich verbunden ist und durch eine äußere Kraft passiv gebogen wird,
die Schlauchbereiche (9) jeweils mit dem zweiten Biegebereich verbunden und so ausgebildet sind, dass sie eine größere Steifigkeit als die Steifigkeit des zweiten Biegebereichs aufweisen, und
bei der Mehrzahl von Endoskopen ein Verhältnis zwischen Längen (L1, L2, L3) der zweiten Biegebereiche so festgelegt ist, dass es einem Größenverhältnis zwischen den Außendurchmessern (d1, d2, d3) der Schlauchbereiche in den Einführbereichen entgegengesetzt ist.

2. Endoskopsystem nach Anspruch 1, wobei Längen der ersten Biegebereiche der Mehrzahl von Endoskopen im Wesentlichen übereinstimmen.

3. Endoskopsystem nach Anspruch 1, wobei Längen der gesamten Biegebereiche einschließlich der ersten Biegebereiche und der zweiten Biegebereiche der Mehrzahl von Endoskopen im Wesentlichen übereinstimmen.

4. Endoskopsystem nach einem beliebigen der Ansprüche 1 bis 3, wobei bei den zweiten Biegebereichen ein Größenverhältnis zwischen maximalen Biegewinkeln so festgelegt ist, dass es einem Größenverhältnis zwischen Durchmessern der Einführbereiche entgegengesetzt ist.

5. Endoskopsystem nach einem beliebigen der Ansprüche 1 bis 4, wobei bei den zweiten Biegebereichen Bögen, die sich bei einer maximalen Biegung durch Außenumfangsbereiche an Außenseiten ergeben, auf im Wesentlichen selbe Krümmungen festgelegt sind.

6. Endoskopsystem nach Anspruch 1, wobei ein Außendurchmesser des zweiten Biegebereichs im Wesentlichen mit dem Außendurchmesser des Schlauchbereichs übereinstimmt.

7. Endoskopsystem nach Anspruch 1, wobei ein Außendurchmesser des ersten Biegebereichs im Wesentlichen mit dem Außendurchmesser des Schlauchbereichs übereinstimmt.

## Revendications

1. Système endoscopique comprenant une pluralité d'endoscopes incluant des parties d'insertion configurées par des parties (7, 8) de flexion et des parties (9) de tubes flexibles, des diamètres extérieurs d'au moins les tubes flexibles étant différents les uns des autres, dans lequel
chacune des parties de flexion dans la pluralité d'endoscopes inclut :
une première partie (7) de flexion soumise à une opération de fléchissement pour fléchir activement ; et
une deuxième partie (8) de flexion connectée à la première partie de flexion et fléchie passivement par une force externe,
les parties (9) de tubes flexibles sont respectivement connectées à la deuxième partie de flexion et formées pour avoir une rigidité supérieure à la rigidité de la deuxième partie de flexion, et
dans la pluralité d'endoscopes, une relation entre des longueurs (L1, L2, L3) des deuxièmes parties de flexion est fixée pour être contraire à une relation de tailles entre les diamètres extérieurs (d1, d2, d3) des parties de tubes flexibles dans les parties d'insertion.

2. Système endoscopique selon la revendication 1, dans lequel des longueurs des premières parties de flexion de la pluralité d'endoscopes sont sensiblement les mêmes.

3. Système endoscopique selon la revendication 1, dans lequel des longueurs de la totalité des parties de flexion incluant les premières parties de flexion et les deuxièmes parties de flexion de la pluralité d'endoscopes sont sensiblement les mêmes.

4. Système endoscopique selon l'une quelconque des revendications 1 à 3, dans lequel, dans les deuxièmes parties de flexion, une relation de tailles entre des angles de flexion maximum est fixée pour être contraire à une relation de tailles entre des diamètres des parties d'insertion.

5. Système endoscopique selon l'une quelconque des revendications 1 à 4, dans lequel, dans les deuxièmes parties de flexion, des arcs de cercle tirés par des parties périphériques extérieures sur des côtés extérieurs lors d'une flexion maximum sont fixés à sensiblement des mêmes courbures.

6. Système endoscopique selon la revendication 1, dans lequel un diamètre extérieur de la deuxième partie de flexion est sensiblement égal au diamètre extérieur de la partie de tube flexible.

7. Système endoscopique selon la revendication 1, dans lequel un diamètre extérieur de la première partie de flexion est sensiblement égal au diamètre extérieur de la partie de tube flexible.
